# EUROPEAN PATENT SPECIFICATION

(11) **EP 1 973 485 B1**
(45) Date of publication and mention of the grant of the patent: **27.11.2013**
(21) Application number: 06787218.4
(22) Date of filing: 13.07.2006
(51) Int. Cl.: A61B 18/18, A61B 18/02

(54) **CRYOGENIC APPLICATOR FOR SKIN REJUVENATING TREATMENT**
KRYOGENER APPLIKATOR ZUR VERJÜNGUNGSBEHANDLUNG DER HAUT
APPLICATEUR CRYOGENIQUE POUR TRAITEMENT ANTI-VIEILLISSEMENT DE LA PEAU

(30) Priority: 06.01.2006 US 757109 P; 11.05.2006 US 432244
(43) Date of publication of application: 01.10.2008
(73) Proprietor: Goulko, Olga, Fort Lee NJ 07024 (US)
(72) Inventor: Goulko, Olga, Fort Lee NJ 07024 (US)
(74) Representative: Kietzmann, Lutz
(86) International application number: PCT/US2006/027278
(87) International publication number: WO 2007/081400

(56) References cited:
- EP-A2- 0 375 579
- EP-B1- 1 196 215
- US-A- 3 103 690
- US-A- 3 869 338
- US-A- 4 032 305
- US-A- 5 516 505
- US-A- 5 738 682
- US-A1- 2003 100 936
- US-A1- 2004 184 864
- US-A1- 2005 043 723
- US-B1- 6 551 309

## Description

### 1. Background of the Invention.

### A. Field of the Invention.

The present invention relates to a cryogenic applicator and, more particularly, to a cryogenic applicator arrangement for rejuvenating skin.

### B. Description of the Prior Art.

Liquid nitrogen, and/or other biocompatible non-toxic cryogenic liquids, all herein sometimes referred to as "cryogenic liquids" is frequently used at offices of physicians in removal of warts, lesions, sun damage and/or the like from a person's skin. For example a method used for removing a wart is to apply liquid nitrogen thereto for a substantial length of time, usually a matter of seconds. The liquid nitrogen has a boiling temperature of approximately -335°F.

Although liquid nitrogen is here mentioned, it will be understood that other suitable biocompatible non-toxic cryogenic liquids could be substituted therefor and the very cold temperature used in the treatment could be different and might vary. In treating the wart, the nitrogen "burns" by freezing the wart.

Numerous innovations for skin rejuvenation have been provided in the prior art that will be described below. Even though each of these innovations may be suitable for a specific purpose to which it is addressed, said innovations all differ in structure and/or technique and/or objective from that of the present invention.

### (1) United States Patent Number 4,074,717 to Schulze et al.

United States Patent Number 4,074,717, which issued to Schulze et al. on February 21, 1978, teaches a cryogenic probe, its method of charging, and its method of use. The probe includes a barrel having a plunger mechanism movably mounted therein and a valve on the lower end thereof. The valve normally closes the lower end of the barrel, but the plunger may be moved relative to the barrel to permit cryogenic liquid, such as liquid nitrogen, to by-pass the valve and move upwardly into the interior of the barrel. The valve has a tip portion at the lower end thereof. The barrel is removably positioned in a guard, so that the tip portion extends outwardly through the bottom of the guard whereby the upper end of the plunger is exposed above the guard. The tip portion has a lower end portion which is extremely thin, so that the cryogenic liquid will be positioned closely adjacent the skin, but not in actual contact therewith when the tip portion is placed into contact with the patient's skin.

### (2) United States Patent Number 5,330, 745 to McDow.

United States Patent Number 5,330,745 issued to McDow on July 19, 1994 and it teaches a method for cryogenically treating a skin lesion employing a hollow fluid retaining device for retaining cryogenic refrigerant in a liquid pool, and then contacting the area of the skin lesion at a temperature and for a time, so that permanent, irreversible rupture of the cellular membrane of the lesion cells occurs.

### (3) United States Patent Number 6,350,276 to Knowlton.

United States Patent Number 6,350,276 issued to Knowlton on February 26, 2002 and it teaches a fluid delivery apparatus for introducing a fluid cooling media to a skin surface, including a template with a skin interface surface. An energy delivery device is coupled to the template. A fluid cooling media introduction member is coupled to the template.

Resources controllably deliver energy from the energy delivery device to the skin surface. In a related embodiment, the resources are configured to controllably deliver the flowable cooling media to the introduction member. In another embodiment, a sensor is coupled to the resources and to the skin surface.

### (4) United States Patent Number 6,726,693 to Weber et al.

United States Patent Number 6,726,693 issued to Weber et al. on April 27, 2004 and it teaches tissue resurfacing accomplished by propelling biocompatible, non-toxic materials at the tissue with sufficient velocity to cause destruction or loosening of tissues to a desired depth. The biocompatible materials are generated by abrading a solid frozen unit and propelling the abraded material onto the surface of the skin or tissue to be treated. A vacuum line near the delivery tip may be used to remove excess materials or reaction by-products building up on the surface of the skin. The treatment system generally includes a control unit, a handheld particle generator, and a cable connecting the control unit to the particle generator. The control unit can include user controls to select particle temperature, particle flux, particle velocity, and vacuum. The handheld particle generator contains a mechanism to push the frozen biocompatible material against a rotating grinding wheel producing the small particles being propelled against tissue being treated.

### (5) United States Patent Number 6,749,624 to Knowlton.

United States Patent Number 6,749,624 issued to Knowlton on June 15, 2004 and it teaches a fluid delivery apparatus for introducing a fluid cooling media to a skin surface, including a template with a skin interface surface. An energy delivery device is coupled to the template. A fluid cooling media introduction member is coupled to the template. Resources controllably deliver energy from the energy delivery device to the skin surface. In a related embodiment, the resources are configured to controllably deliver the flowable cooling media to the introduction member. In another embodiment, a sensor is coupled to the resources and to the skin surface.

### (6) United States Patent Number 6, 764,493 to Weber et al.

United States Patent Number 6,764,493 issued to Weber et al. on July 20, 2004 teaches biocompatible materials being propelled at the skin with sufficient velocity to cause desired resurfacing of tissue to the desired penetration depth. The materials, such as dry ice or water ice, are harmonious with the human body and thus eliminate foreign body reactions. Various materials may be used in combination, including local anesthetics and vasoconstrictors in solid or liquid form. The biocompatible solid or liquid particles are suspended in a cold carrier fluid and are propelled through an insulated delivery system to the surface of the skin. The treatment of diseased skin lesions may be accomplished by its use as a drug delivery system.

### (7) United States Patent Number 5738682 to Jensma.

United States Patent Number 5738682 to Jensma issued on April 14, 1998 teaches an apparatus for cooling surfaces by evaporating a liquid refrigerant. The apparatus comprises a container with a liquid refrigerant, a feed tube, an outlet tube and a dispensing head. The dispensing head comprises an open celled foam secured to the outlet tube. This known assembly is used for cooling the skin of a human being, especially for freezing warts and the like, so that they can be removed.

It is apparent that numerous innovations for skin treatments have been provided in the prior art. Even though these innovations each may be suitable for a specific purpose to which it is addressed, the innovations would not be suitable, either individually or collectively, for the purposes of the present invention as hereafter described.

### 3. Summary of the Invention.

A general objective of the present invention is to provide a biocompatible non-toxic cryogenic fluid applicator for rejuvenating skin to overcome disadvantages of the prior art.

The invention is defined in the independent claim 1. Preferred embodiments of the invention are defined in the appended dependent claims.

A barrel is hand-held. A head is rotatably mounted to the barrel. A cryogenic interface fluidly connects the barrel to a source of a biocompatible non-toxic cryogenic fluid to supply the biocompatible non-toxic cryogenic fluid through the barrel to the head that in turn sparges the biocompatible non-toxic cryogenic fluid onto the skin quickly, evenly, and smoothly when the head is rolled on the skin, and thereby rejuvenate the skin.

The novel features which are considered characteristic of the present invention are set forth in the appended claims. The invention itself, however, both as to its construction and its method of operation, together with additional objects and advantages thereof, will be best understood from the following description of the specific embodiments when read and understood in connection with the accompanying drawing.

### 4. Brief Description of the Drawing.

The figures of the drawing are briefly described as follows:
- **FIGURE 1**: is a diagrammatic perspective view of the cryogenic applicator according to an embodiment of the present invention for rejuvenating skin on a person's forehead;
- **FIGURE 2**: is an enlarged diagrammatic perspective view of the cryogenic applicator of the present invention identified by **ARROW 2** in **FIGURE 1****;**
- **FIGURE 3**: is an enlarged diagrammatic cross sectional view taken along **LINE 3-3 in** **FIGURE 2****;**
- **FIGURE 4**: is an enlarged diagrammatic cross sectional view of the area generally enclosed by the dotted curve identified by **ARROW 4** in **FIGURE 3****;**
- **FIGURE 5**: is an enlarged diagrammatic cross sectional view of the area generally enclosed by the dotted curve identified by **ARROW 5** in **FIGURE 3****;**
- **FIGURE 6**: is an enlarged diagrammatic cross sectional view of the area generally enclosed by the dotted curve identified by **ARROW 6** in **FIGURE 3****;** and
- **FIGURES 7A-7B**: is a flowchart sequencing steps in rejeuvenation of the skin utilizing the cryogenic applicator of the present invention.

### 5. List of Reference Numerals Utilized in the Drawing.

- **10**: cryogenic applicator of present invention for rejuvenating skin **12**
- **12**: skin
- **14**: barrel
- **15**: flow-control dial
- **16**: head
- **18**: cryogenic interface (such as a supple tube) for fluidly connecting barrel **14** to a source **22** of cryogenic fluid **20** to supply cryogenic fluid **20** through barrel **14** to head **16** that in turn sparges cryogenic fluid **20** onto skin **12** quickly, evenly, and smoothly when head **16** is rolled on skin **12,** and thereby rejuvenating skin **12**
- **20**: cryogenic fluid
- **22**: source of cryogenic fluid **20**
- **23**: interior of barrel **14** for directing biocompatible non-toxic cryogenic liquid **20** to head **16**
- **24**: proximal portion of barrel **14**
- **26**: distal portion of barrel **14**
- **28**: handle of proximal portion **24** of barrel **14** for being hand-held
- **30**: plurality of openings extending radially through and spaced axially along distal portion **26** of barrel **14** and communicating with interior **23** of barrel **14** for passing cryogenic liquid **20** to head **16**
- **32**: circumferential groove extending completely around barrel **14**
- **34**: end of distal portion **26** of barrel **14**
- **36**: tit extending axially outwardly from end **34** of distal portion **26** of barrel **14**
- **38**: flexible supply tube of cryogenic interface **18** for fluidly communicating with source **22** of cryogenic fluid **20** to supply cryogenic fluid **20** through interior **23** of barrel **14,** out through plurality of openings **30** in distal portion **26** of barrel **14,** and onto head **16** that in turn sparges cryogenic fluid **20** onto skin **12** quickly, evenly, and smoothly when head **16** is rolled on skin **12,** and thereby rejuvenating skin **12**
- **39**: valve for regulating flow of cryogenic fluid
- **40**: drum of head **16**
- **42**: web of head **16**
- **44**: mesh of drum **40** of head **16**
- **46**: soft porous material of web **42** of head **16** for contacting skin **12** in rolling action
- **48**: proximal end of mesh **44** of drum **40** of head **14**
- **50**: distal end of mesh **44** of drum **40** of head **14**
- **52**: circumferential ring of proximal end **48** of mesh **44** of drum **40** of head **14**
- **54**: opening in distal end **50** of mesh **44** of drum **40** of head **14**

### 6. Detailed Description of the Preferred Embodiment.

### A. General.

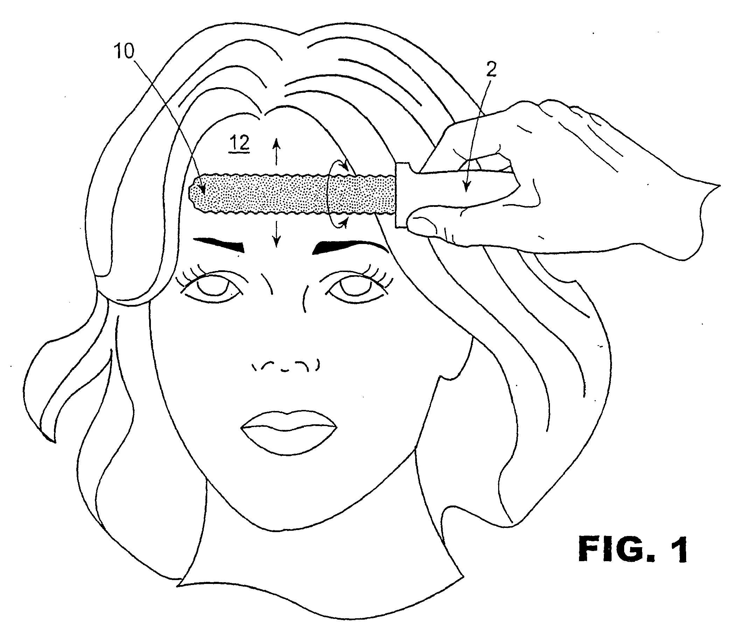

Referring now to the figures, in which like numerals indicate like parts, and particularly to **FIGURE 1****,** which is a diagrammatic perspective view of the cryogenic applicator of the present invention rejuvenating skin (for example) on the forehead, the cryogenic applicator of the present invention is shown generally at **10** for rejuvenating skin **12.**

### B. Overall Configuration.

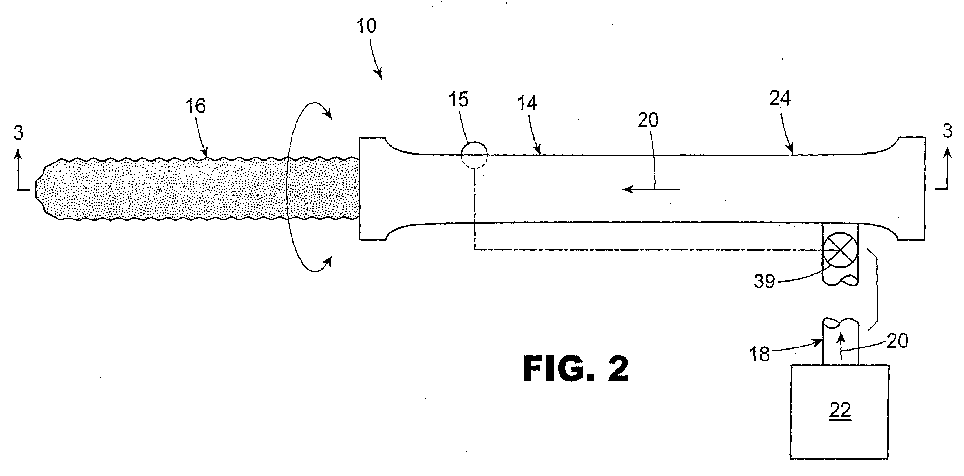

The overall configuration of the cryogenic applicator **10** can best be seen in **FIGURE 2****,** which an enlarged diagrammatic perspective view of the cryogenic applicator of the present invention identified by **ARROW 2 in** **FIGURE 1****,** and as such, will be discussed in conjunction therewith.

The cryogenic applicator **10** comprises a barrel **14**, a head **16,** and a cryogenic interface **18.** The barrel **14** is hand-held and is provided with a flow-control dial **15.** The head **16** is rotatably mounted to the barrel **14.** The cryogenic interface **18** is for fluidly connecting the barrel **14** to a source **22** of a biocompatible non-toxic cryogenic fluid **20** to supply the cryogenic fluid **20** through the barrel **14** to the head **16** that in turn sparges the biocompatible non-toxic cryogenic fluid **20** onto the skin **12** quickly, evenly, and smoothly when the head **16** is rolled on the skin **12,** and thereby rejuvenating the skin **12.** The valve 39 or similar apparatus for controlling flow of the cryogenic liquid can be arranged, as is well known in the art at the source **22,** in the interface **18** or at the barrel **14.** The valve 39 or similar apparatus is controllable by the operator by means of the dial 15 using means also well known in the art.

The cryogenic applicator **10** provides a simple, effective means for wrinkle, lesion, and discoloration reduction or elimination thereof from the skin **12.** Applicant achieves rejuvenation of the skin **12** by using a quick, smooth, and even application of the cryogenic fluid **20,** *e.g.,* liquid nitrogen or other suitable biocompatible non-toxic cryogenic liquid, over one or more substantial surfaced areas of a person's skin **12.** Typically the areas of the skin **12** treated include the forehead, the temples, the nose, the cheeks, around the eyes, the cheeks, the chin, the neck, the backs of the hands, and/or other suitable areas for a dramatically shorter time period, typically in the order of hundredths or tenths of a second, so that the cryogenic liquid **20** quickly evaporates, wrinkles, lesions and discolorations are reduced or eliminated, and the skin **12** is rejuvenated. These things happen while the person experiences mild exhilaration. The treatment is painless, eliminating a need for local anesthetic, in fact it is a pleasurable experience, It affects only the epidermal layer(s) of the skin **12** because the cryogenic liquid **12** is applied at its boiling temperature for a very short length of time, so that damage to the epidermal layer does not occur.

With proper operation of the cryogenic applicator **10,** heat of the skin **12** very quickly evaporates the cryogenic liquid **20** in at the most one to two seconds to treat wrinkles, blemishes, and/or discolorations without adversely affecting the skin **12.** Waste of the biocompatible non-toxic cryogenic liquid **20** is avoided eliminating a need for expensive facilities for storage thereof. The cryogenic applicator **10** is economical to manufacture, reliable, durable, easy and safe to use, refined in appearance, easy to clean, and easy to maintain.

### C. Specific Configuration.

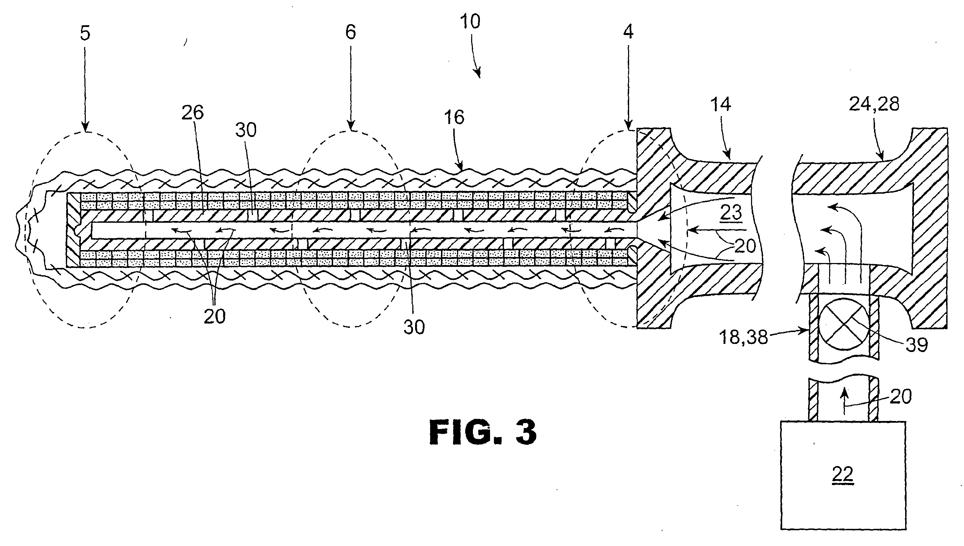

The specific configuration of the barrel **14,** the head **16,** and the cryogenic interface **18** can best be seen in **FIGURES 3-6**, which are, respectively, an enlarged diagrammatic cross sectional view taken along **LINE 3-3** in **FIGURE 2****,** an enlarged diagrammatic cross sectional view of the area generally enclosed by the dotted curve identified by **ARROW 4** in **FIGURE 3****,** an enlarged diagrammatic cross sectional view of the area generally enclosed by the dotted curve identified by **ARROW 5** in **FIGURE 3****,** and an enlarged diagrammatic cross sectional view of the area generally enclosed by the dotted curve identified by **ARROW 6** in **FIGURE 3****,** and as such, will be discussed with reference thereto.

The **barrel 14** is slender and elongated and has a hollow interior **23,** a proximal portion **24,** and a distal portion **26**. The hollow interior **23** of the barrel **14** is for directing the biocompatible non-toxic cryogenic liquid **20** to the head **16.** The proximal portion **24** of the barrel **14** functions as a handle **28** for being hand-held. The distal portion **26** of the barrel **14** has a plurality of openings **30** extending radially therethrough, spaced axially therealong, and communicating with the hollow interior **23** of the barrel **14** for passing the biocompatible non-toxic cryogenic liquid **20** to the head **16.** The source of the cryogenic fluid **20** could be arranged optionally in the barrel **14.** By this expedient the interface (supply tube) **18** could be eliminated. The barrel **14** could be thinner and ergonomically shaped, being thinner or wider in its middle. The barrel **14** should heft like a knife handle.

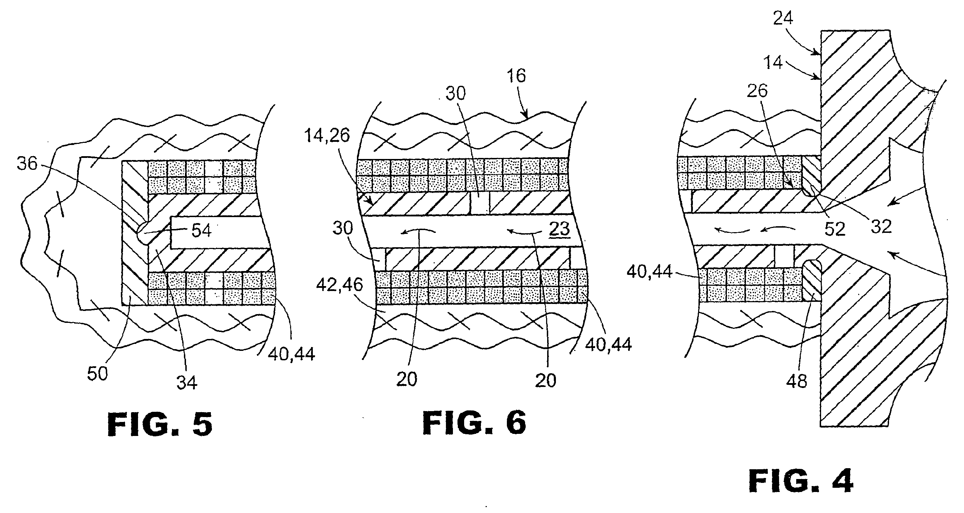

As shown in **FIGURE 4****,** the barrel **14** further has a circumferential groove **32** extending completely therearound and being located where the proximal portion **24** of the barrel **14** meets the distal portion **26** of the barrel **14.**

As shown in **FIGURE 5****,** the distal portion **26** of the barrel **14** terminates in an end **34.** The end **34** of the distal portion **26** of the barrel **14** is closed and has a tit **36** extending axially outwardly therefrom.

Returning now to **FIGURE 3****,** the cryogenic interface **18** is a flexible tube **38** fluidly communicating with the proximal portion **24** of the barrel **14** and for fluidly communicating with the source **22** of the cryogenic fluid **20** to supply the cryogenic fluid **20** through the hollow interior **23** of the barrel **14,** out through the plurality of openings **30** in the distal portion **26** of the barrel **14,** and onto the head **16 (****FIG. 6****)** that in turn sparges the cryogenic fluid **20** onto the skin **12** quickly, evenly, and smoothly when the head **16** is rolled on the skin **12,** and thereby rejuvenating the skin **12.**

As shown in **FIGURE 6****,** the head **16** comprises a drum **40** and a web **42.** The drum **40** of the head **16** is open surfaced, such as a mesh **44,** and is generally cylindrically-shaped and rotatably mounted to the distal portion **26** of the barrel **14.** The web **42** of the head **16** is a soft porous material **46,** such as cotton or fabric, woven or non-woven, overlying the mesh **44** of the drum **40** of the head **16** and affixed thereto for contacting the skin **12** in a rolling action. The mesh **44** can be replacably disposable like a sock.

The mesh **44** of the drum **40** of the head **16** provides support for the soft porous material **46** of the web **42** of the head **16,** while allowing the cryogenic fluid **20** passing through the plurality of openings **30** in the distal portion **26** of the barrel **14** to pass therethrough and into the soft porous material **46** of the web **42** of the head **16** that in turn sparges the cryogenic fluid **20** onto the skin **12** quickly, evenly, and smoothly when the head **16** is rolled on the skin **12,** and thereby rejuvenating the skin **12.**

The mesh **44** of the drum **40** of the head **16** has a proximal end **48 (****FIG. 4****)** and a distal end **50 (****FIG. 5****).** As shown in **FIGURE 4****,** the proximal end **48** of the mesh **44** of the drum **40** of the head **16** is formed into a circumferential ring **52** rotatably engaging in the circumferential groove **32** in the barrel **14,** and as shown in **FIGURE 5****,** the distal end **50** of the mesh **44** of the drum **40** of the head **16** has an opening **54** therein rotatably receiving the tit **36** on the end **34** of the distal portion **26** of the barrel **14,** for journaling and allowing the head **16** to rotate axially relative to the barrel **14.**

It is to be understood that the positioning of the opening **54** and the tit **36** can be reversed.

### D. Treatment.

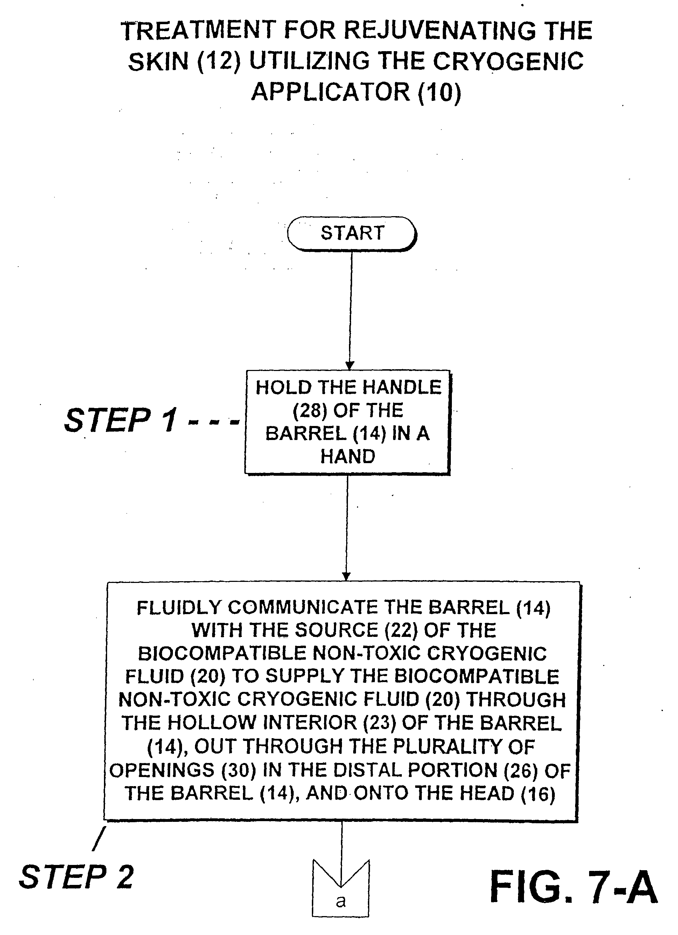

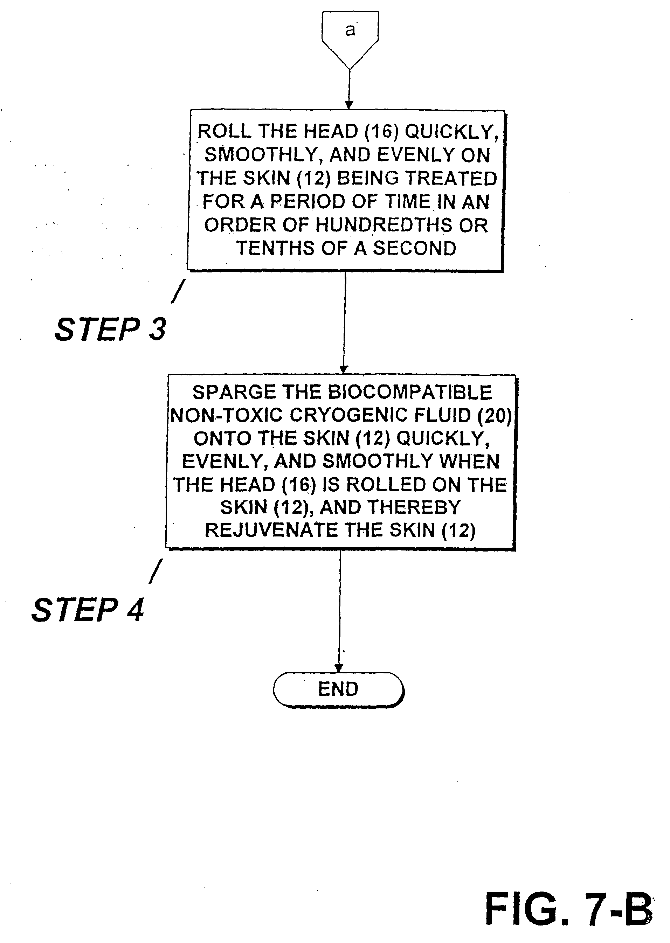

The treatment for rejuvenating skin **12** utilizing the cryogenic applicator **10** can best be seen **FIGURES 7A-7B**, which, taken together, are a flowchart sequencing steps for rejuvenating the skin utilizing the cryogenic applicator of the present invention, and as such, will be discussed with reference thereto.
- ***STEP 1:***: Hold the handle **28** of the barrel **14** in a hand. The dial **15** will be used to control flow of cryogenic fluid **20** using valve **39..**
- ***STEP 2:***: Fluidly communicate the barrel with the source **22** of the cryogenic fluid **20** to supply the cryogenic fluid **20** through the interior **23** of the barrel **14,** out through the plurality of openings **30** in the distal portion **26** of the barrel **14,** and onto the head 16. Control flow of the cryogenic material (if necessary) by means of the dial **15.**
- ***STEP** 3:*: Roll the head **16** quickly, smoothly, and evenly over the skin **12** being treated for a period of time of a different magnitude from typical procedures, such as "burning" of warts, more in the order of hundredths or tenths of a second.
- ***STEP 4:***: Sparge the biocompatible non-toxic cryogenic fluid **20** onto the skin **12** quickly, evenly, and smoothly when the head **16** is rolled on the skin **12,** and thereby rejuvenate the skin **12.**

The person experiences a mild tingling and exhilarating feeling. Wrinkles, blemishes, and discolorations are reduced or eliminated. The skin **12** is stimulated and rejuvenated. The old look younger, the young look better, all look happier, their skin **12** looks refreshed, and they feel buoyant. So their usual reaction is to rejoice.

It is to be understood that some other biocompatible non-toxic cryogenic fluid **20** could be used in place of the liquid nitrogen and the form of the handle **28** of the proximal portion **24** of the barrel **14** could be modified. Connection with the supply **22** of the cryogenic fluid **20** and its control **15** and valve **39** could take different forms. Methods of connecting the head **16** mechanically to the barrel **14** for axial rotation thereabout may vary. The materials of the barrel **14** and the head **16** could be either metal, plastic, or some combination thereof, and the cotton of the soft porous material **46** might be replaced typically by a non-woven fabric or any other suitable soft porous material.

## Claims

1. A cryogenic applicator arrangement (10) for rejuvenating skin, comprising a barrel (14), a head (16) and a cryogenic interface (18), wherein said barrel (14) is hand-held, slender and elongated, wherein said head (16) is rotatably mounted to said barrel (14),wherein the cryogenic interface (18) is for fluidly connecting said barrel (14) to a source (22) of a biocompatible non-toxic cryogenic fluid (20),
wherein the biocompatible non-toxic cryogenic fluid (20) is supplied through said barrel (14) to said head (16) that in turn is configured to sparge the biocompatible non-toxic cryogenic fluid (20) onto the skin (12) quickly, evenly, and smoothly when said head (16) is rolled on the skin (12) and thereby rejuvenating the skin (12), wherein said barrel (14) has:
a hollow interior (23) for directing the biocompatible non-toxic cryogenic fluid (20) to said head (16),
a flow-control dial (15) for controlling a valve (39) for regulating flow of the cryogenic fluid (20),
a proximal portion (24), which functions as a handle (28) for being handheld, and
a distal portion (26) with a plurality of openings (30) extending radially through the distal portion (26) and being spaced axially along the distal portion (26) to communicate with said hollow interior (23) for passing the biocompatible non-toxic cryogenic fluid (20) to said head (16), said head being rotatably mounted to said distal portion of the barrel.

2. The applicator arrangement (10) of claim 1, wherein said barrel (14) has a circumferential groove (32), which
extends completely said barrel and
is located where said proximal portion (24) of said barrel (14) meets said distal portion (26) of said barrel (14).

3. The applicator arrangement (10) of claim 2, wherein said distal portion (26) of said barrel (14) terminates in a closed end (34), said closed end (34) having
a tit (36); and wherein said tit (36) of said distal portion (26) of said barrel (14) extends axially outwardly therefrom.

4. The applicator arrangement (10) of claim 1, wherein said cryogenic interface (18) is a flexible tube (38); and
wherein said flexible tube (38) of said cryogenic interface (18) fluidly communicates with said proximal portion (24) of said barrel (14) and for fluidly communicating with the source (22) of the biocompatible non-toxic cryogenic fluid (20) to supply the biocompatible non-toxic cryogenic fluid (20) through said hollow interior (23) of said barrel (14), out through said plurality of openings (30) in said distal portion (26) of said barrel (14).

5. The applicator arrangement (10) of claim 3, wherein said head (16) comprises a drum (40) and a web (42).

6. The applicator arrangement (10) of claim 5, wherein said drum (40) of said head (16) is open surfaced and generally cylindrically-shaped; and
wherein said drum (40) of said head (16) is rotatably mounted to said distal portion (26) of said barrel (14).

7. The applicator arrangement (10) of claim 5, wherein said drum (40) of said head (16) is mesh (44).

8. The applicator arrangement (10) of claim 7, wherein said web (42) of said head (16) is a soft porous material (46), which
overlies and is affixed to said mesh (44) of said drum (40) of said head (16);
and
wherein said soft porous material (46) of said web (42) of said head (16) is for contacting the skin (12) in a rolling action.

9. The applicator arrangement (10) of claim 8, wherein said soft porous material (46) of said web (42) of said head (16) is selected from the group consisting of cotton, fabric, woven, and non-woven.

10. The applicator arrangement (10) of claim 8, wherein said mesh (44) of said drum (40) of said head (16) provides support for said soft porous material (46) of said web (42) of said head (16), while allowing the biocompatible nontoxic cryogenic fluid (20) passing through said plurality of openings (30) in said distal portion (26) of said barrel (14) to pass therethrough and into said soft porous material (46) of said web (42) of said head (16).

11. The applicator arrangement (10) of claim 7, wherein said mesh (44) of said drum (40) of said head (16) has a proximal end (48) and
a distal end (50);
wherein said proximal end (48) of said mesh (44) of said drum (40) of said head (16) is formed into a circumferential ring (52), which
rotatably engages in said circumferential grove (32) in said barrel (14) and said distal end (50) of said mesh (44) has an opening (54) therein rotatably receiving said tit (36) on said end (34) of said distal portion (26) of said barrel (14), thereby journaling and allowing said head (16) to rotate axially relative to said barrel (14).

12. The applicator arrangement (10) of claim 5, wherein said barrel (14) is made from a material selected from the group consisting of metal, plastic, and a combination thereof; and
wherein said drum (40) of said head (16) is made from a material selected from the group consisting of metal, plastic, and a combination thereof.

13. The applicator arrangement (10) of claim 7, wherein said distal portion (26) of said barrel (14) terminates in an end (34) having an opening (54) therein.

14. The applicator arrangement (10) of claim 13, wherein said mesh (44) of said drum (40) of said head (16) has a distal end (50) having a tit (36) extending axially inwardly therefrom being rotatably received in an opening (54) in said end (34) of said distal portion (26) of said barrel (14), thereby journaling and allowing said head (16) to rotate axially relative to said barrel (14).

15. The applicator arrangement (10) of claim 1, wherein the biocompatible non-toxic cryogenic fluid (20) is liquid nitrogen.

## Patentansprüche

1. Kryogene Applikatoranordnung (10) zum Regenerieren von Haut, die einen Schaft (14), einen Kopfteil (16) und eine kryogene Grenzfläche (18) umfasst, wobei der Schaft (14) in der Hand gehalten wird, schlank und langgestreckt ist, wobei das Kopfteil (16) drehbar am Schaft (14) befestigt ist, wobei die kryogene Grenzfläche (18) der Fluidverbindung des Schaftes (14) mit einer Quelle (22) eines biokompatiblen, nicht toxischen, kryogenen Fluids (20) dient, wobei das biokompatible, nicht toxische, kryogene Fluid (20) durch den Schaft (14) dem Kopfteil (16) zugeführt wird, das wiederum dafür ausgelegt ist, das biokompatible, nicht toxische, kryogene Fluid (20) schnell, gleichmäßig und sanft auf die Haut (12) aufzubringen, wenn das Kopfteil (16) auf der Haut (12) gerollt wird, um dadurch die Haut (12) zu regenerieren, wobei der Schaft (14) Folgendes aufweist:
ein hohles Inneres (23) zum Lenken des biokompatiblen, nicht toxischen, kryogenen Fluids (20) zum Kopfteil (16),
einen Durchflussregler (15) zum Steuern eines Ventils (39) zum Regulieren des Durchflusses des kryogenen Fluids (20),
einen proximalen Abschnitt (24), der als Handgriff (28) zum Halten in der Hand fungiert,
und
einen distalen Abschnitt (26) mit mehreren Öffnungen (30), die sich radial durch den distalen Abschnitt (26) erstrecken und axial entlang des distalen Abschnitts (26) verteilt sind, um mit dem hohlen Inneren (23) zum Durchleiten des biokompatiblen, nicht toxischen, kryogenen Fluids (20) zum Kopfteil (16) zu kommunizieren, wobei das Kopfteil drehbar am distalen Abschnitt des Schafts befestigt ist.

2. Applikatoranordnung (10) nach Anspruch 1, wobei der Schaft (14) eine Umfangsnut (32) aufweist, die sich vollständig um den Schaft herum erstreckt und sich dort befindet, wo der proximale Abschnitt (24) des Schaftes (14) auf den distalen Abschnitt (26) des Schaftes (14) trifft.

3. Applikatoranordnung (10) nach Anspruch 2, wobei der distale Abschnitt (26) des Schaftes (14) in einem geschlossenen Ende (34) endet, wobei das geschlossene Ende (34) in einem Nippel (36) endet, und wobei der Nippel (36) des distalen Abschnitts (26) des Schaftes (14) sich von dort aus axial nach außen erstreckt.

4. Applikatoranordnung (10) nach Anspruch 1, wobei die kryogene Grenzfläche (18) ein flexibles Rohr (38) ist; und
wobei das flexible Rohr (38) der kryogenen Grenzfläche (18) per Fluid mit dem proximalen Abschnitt (24) des Schaftes (14) kommuniziert und per Fluid mit der Quelle (22) des biokompatiblen, nicht toxischen, kryogenen Fluids (20) kommuniziert, um das biokompatible, nicht toxische, kryogene Fluid (20) durch das hohle Innere (23) des Schaftes (14) zuzuführen, nach außen durch die mehreren Öffnungen (30) des distalen Abschnitts (26) des Schaftes (14).

5. Applikatoranordnung (10) nach Anspruch 3, wobei der Kopfteil (16) eine Trommel (40) und ein Netz (42) umfasst.

6. Applikatoranordnung (10) nach Anspruch 5, wobei die Trommel (40) des Kopfteils (16) eine offene Oberfläche aufweist und Im Allgemeinen zylindrisch geformt ist; und
wobei die Trommel (40) des Kopfteils (16) drehbar am distalen Abschnitt (26) des Schaftes (14) befestigt ist.

7. Applikatoranordnung (10) nach Anspruch 5, wobei die Trommel (40) des Kopfteils (16) ein Geflecht (44) ist.

8. Applikatoranordnung (10) nach Anspruch 7, wobei das Netz (42) des Kopfteils (16) ein weiches, poröses Material (46) ist, das über dem Geflecht (44) der Trommel (40) des Kopfteils (16) liegt und an demselben befestigt ist;
wobei das weiche, poröse Material (46) des Netzes (42) des Kopfteils (16) zum Herstellen eines Kontaktes mit der Haut (12) in einer rollenden Weise dient.

9. Applikatoranordnung (10) nach Anspruch 8, wobei das weiche, poröse Material (46) des Netzes (42) des Kopfteils (16) aus der Gruppe bestehend aus Baumwolle, Stoff, gewebt und nicht gewebt, auswählbar ist.

10. Applikatoranordnung (10) nach Anspruch 8, wobei das Geflecht (44) der Trommel (40) des Kopfteils (16) für eine Abstützung für das weiche, poröse Material (46) des Netzes (42) des Kopfteils (16) sorgt, während es dem biokompatiblen, nicht toxischen, kryogenen Fluid (20) ermöglicht, durch die mehreren Öffnungen (30) im distalen Abschnitt (26) des Schaftes (14) zu strömen, um durch dasselbe und in das weiche, poröse Material (46) des Netzes (42) des Kopfteils (16) zu fließen.

11. Applikatoranordnung (10) nach Anspruch 7, wobei das Geflecht (44) der Trommel (40) des Kopfteils (16) ein proximales Ende (48) und ein distales Ende (50) aufweist;
wobei das proximale Ende (48) des Geflechts (44) der Trommel (40) des Kopfteils (16) zu einem Umfangsring (52) geformt ist, der drehbar in die Umfangsnut (32) im Schaft (14) eingreift und das distale Ende (50) des Geflechts (44) eine Öffnung (54) darin hat, die drehbar den Nippel (36) am Ende (34) des distalen Abschnitts (26) des Schafts (14) aufnimmt, wobei das Kopfteil (16) einen Zapfen aufweist und axial um den Schaft (14) rotieren kann.

12. Applikatoranordnung (10) nach Anspruch 5, wobei der Schaft (14) aus einem Material hergestellt ist, das aus der Gruppe bestehend aus Metall, Kunststoff und einer Kombinationen derselben auswählbar ist; und
wobei die Trommel (40) des Kopfteils (16) aus einem Material hergestellt ist, welches aus der Gruppe bestehend aus Metall, Kunststoff und einer Kombinationen derselben auswählbar ist.

13. Applikatoranordnung (10) nach Anspruch 7, wobei der distale Abschnitt (26) des Schaftes (14) in einem Ende (34) endet, das eine Öffnung (54) darin aufweist.

14. Applikatoranordnung (10) nach Anspruch 13, wobei das Geflecht (44) der Trommel (40) des Kopfteils (16) ein distales Ende (50) mit einem Nippel (36) aufweist, der drehbar in einer Öffnung (54) im Ende (34) des distalen Abschnitts (26) des Schaftes (14) aufgenommen ist, wobei das Kopfteil (16) einen Zapfen aufweist und axial um den Schaft (14) rotieren kann.

15. Applikatoranordnung (10) nach Anspruch 1, wobei das biokompatible, nicht toxische, kryogene Fluid (20) flüssiger Stickstoff ist.

## Revendications

1. Dispositif applicateur cryogénique (10) pour rajeunir la peau, comprenant un cylindre (14), une tête (16) et une interface cryogénique (18), où ledit cylindre (14) est portatif, fin et allongé, où ladite tête (16) est montée de manière pivotable sur ledit cylindre (14), où l'interface cryogénique (18) sert à connecter de manière fluide ledit cylindre (14) à une source (22) d'un liquide cryogénique non-toxique et biocompatible (20),
où le liquide cryogénique non-toxique et biocompatible (20) est alimenté par ledit cylindre (14) vers ladite tête (16) qui à son tour est configurée pour arroser le liquide cryogénique non-toxique et biocompatible (20) sur la peau (12) rapidement, uniformément et doucement quand ladite tête (16) est roulée sur la peau (12) et rajeunir ainsi la peau (12), où ledit cylindre (14) a :
un intérieur creux (23) pour diriger le liquide cryogénique non-toxique et biocompatible (20) vers ladite tête (16),
un cadran de réglage de flux (15) pour contrôler une vanne (39) pour réguler le flux du liquide cryogénique (20),
une partie proximale (24) qui fonctionne comme une poignée (28) pour être portative, et
une partie distale (26) avec une pluralité d'ouvertures (30) se prolongeant radialement par la partie distale (26) et étant espacées axialement le long de la partie distale (26) pour communiquer avec ledit intérieur creux (23) pour faire passer le liquide cryogénique non-toxique et biocompatible (20) vers ladite tête (16), ladite tête étant montée de manière pivotable sur ladite partie distale du cylindre.

2. Dispositif applicateur (10) de la revendication 1, où ledit cylindre (14) a une rainure circonférentielle (32) qui se prolonge complètement autour dudit cylindre et qui est situé là où ladite partie proximale (24) dudit cylindre (14) rencontre ladite partie distale (26) dudit cylindre (14).

3. Dispositif applicateur (10) de la revendication 2, où ladite partie distale (26) dudit cylindre (14) se termine en une extrémité fermée (34), ladite extrémité fermée (34) ayant une protubérance (36) ; et où ladite protubérance (36) de ladite partie distale (26) dudit cylindre (14) se prolonge axialement extérieurement à celui-ci.

4. Dispositif applicateur (10) de la revendication 1, où ladite interface cryogénique (18) est un tube flexible (38) ; et
où ledit tube flexible (38) de ladite interface cryogénique (18) communique de manière fluide avec ladite partie proximale (24) dudit cylindre (14) et communique de manière fluide avec la source (22) du liquide cryogénique non-toxique et biocompatible (20) pour alimenter le liquide cryogénique non-toxique et biocompatible (20) par ledit intérieur creux (23) dudit cylindre (14), par ladite pluralité d'ouvertures (30) dans ladite partie distale (26) dudit cylindre (14).

5. Dispositif applicateur (10) de la revendication 3, où ladite tête (16) comprend un tambour (40) et une toile (42).

6. Dispositif applicateur (10) de la revendication 5, où ledit tambour (40) de ladite tête (16) a une surface ouverte et une forme généralement cylindrique ; et
où ledit tambour (40) de ladite tête (16) est monté de manière pivotable sur ladite partie distale (26) dudit cylindre (14).

7. Dispositif applicateur (10) de la revendication 5, où ledit tambour (40) de ladite tête (16) est un filet (44).

8. Dispositif applicateur (10) de la revendication 7, où ladite toile (42) de ladite tête (16) est un matériau poreux doux (46) qui recouvre et est fixé au filet (44) dudit tambour (40) de ladite tête (16) ; et
où ledit matériau poreux doux (46) de ladite toile (42) de ladite tête (16) sert à faire contact avec la peau (12) dans une action de roulement.

9. Dispositif applicateur (10) de la revendication 8, où ledit matériau poreux doux (46) de ladite toile (42) de ladite tête (16) est sélectionné à partir du groupe comprenant du coton, du tissu, du tissé et du non-tissé.

10. Dispositif applicateur (10) de la revendication 8, où ledit filet (44) dudit tambour (40) de ladite tête (16) assure un support pour ledit matériau poreux doux (46) de ladite toile (42) de ladite tête (16) tout en permettant au liquide cryogénique non-toxique et biocompatible (20) passant par la pluralité d'ouvertures (30) dans ladite partie distale (26) dudit cylindre (14) de passer au travers et dans ledit matériau poreux doux (46) de ladite toile (42) de ladite tête (16).

11. Dispositif applicateur (10) de la revendication 7, où ledit filet (44) dudit tambour (40) de ladite tête (16) a une extrémité proximale (48) et une extrémité distale (50);
où ladite extrémité proximale (48) dudit filet (44) dudit tambour (40) de ladite tête (16) est formée dans un anneau circonférentiel (52) qui s'engage de manière pivotable dans ladite rainure circonférentielle (32) dans ledit cylindre (14) et ladite extrémité distale (50) dudit filet (44) a une ouverture (54) à l'intérieur recevant de manière pivotable ladite protubérance (36) sur ladite extrémité (34) de ladite partie distale (26) dudit cylindre (14), journalisant et permettant ainsi à ladite tête (16) de pivoter axialement par rapport audit cylindre (14).

12. Dispositif applicateur (10) de la revendication 5, où ledit cylindre (14) est fait d'un matériau sélectionné à partir du groupe comportant du métal, du plastique et une combinaison de ceux-ci ; et
où ledit tambour (40) de ladite tête (16) est fait d'un matériau sélectionné à partir du groupe comportant du métal, du plastique et une combinaison de ceux-ci.

13. Dispositif applicateur (10) de la revendication 7, où ladite partie distale (26) dudit cylindre (14) se termine en une extrémité fermée (34), ayant une ouverture (54) à l'intérieur.

14. Dispositif applicateur (10) de la revendication 13, où ledit filet (44) dudit tambour (40) de ladite tête (16) a une extrémité distale (50) ayant une protubérance (36) se prolongeant axialement intérieurement de celle-ci et étant reçue de manière pivotable dans une ouverture (54) dans ladite extrémité (34) de ladite partie distale (26) dudit cylindre (14), journalisant et permettant ainsi à ladite tête (16) de pivoter axialement par rapport audit cylindre (14).

15. Dispositif applicateur (10) de la revendication 1, où le liquide cryogénique non-toxique et biocompatible (20) est de l'azote liquide.
